# EUROPÄISCHE PATENTSCHRIFT

(11) **EP 0 788 355 B1**
(45) Veröffentlichungstag und Bekanntmachung des Hinweises auf die Patenterteilung: **22.09.1999**
(21) Anmeldenummer: 95918533.1
(22) Anmeldetag: 17.05.1995
(51) Int. Cl.: A61K 31/295

(54) **VERWENDUNG VON INTRAVENÖS APPLIZIERBAREM EISEN ZUR THERAPIE VON TUMORERKRANKUNGEN UND/ODER INFEKTIONSKRANKHEITEN**
USE OF INTRAVENOUSLY ADMINISTERED IRON IN THE THERAPY OF TUMORAL AND INFECTIOUS DISEASES
UTILISATION DE FER ADMINISTRABLE PAR VOIE INTRAVEINEUSE POUR LE TRAITEMENT D'AFFECTIONS TUMORALES ET/OU DE MALADIES INFECTIEUSES

(30) Priorität: 01.06.1994 DE 4419256
(43) Veröffentlichungstag der Anmeldung: 13.08.1997
(73) Patentinhaber: Rau Freiherr von Nagell, Helmut, 80797 München (DE)
(72) Erfinder: Rau Freiherr von Nagell, Helmut, 80797 München (DE)
(74) Vertreter: Keller, Günter, Dr.
(86) Internationale Anmeldenummer: DE9500665
(87) Internationale Veröffentlichungsnummer: WO9532711

(56) Entgegenhaltungen:
- EP-A- 0 246 734
- DE-A- 2 659 799
- BR. MED. J., Bd. 1, Nr. 6114, 1978 Seiten 683-685, K. WEINBREN ET AL. 'Intramuscular injections of iron compounds and oncogenesis in man.'
- SCAND. J. HAEMATOL. -SUPPL., Bd. 32, 1977 Seiten 87-98, G. MAGNUSSON ET AL. 'Oncological study in rats of ferastral, an iron-poly(sorbitol-gluconic acid) complex, after intramuscular administration.'
- PERIOD. BIOL., Bd. 87, Nr. 1, 1985 Seiten 17-21, M. POLJAK-BLAZY ET AL 'Inhibitory effect of iron on melanoma B16 growth.'

## Beschreibung

Eisen wird als Therapeutikum seit jeher in allen möglichen Formen, zur Kräftigung, Abwehrsteigerung, bei Blutverlusten etc. eingesetzt und insbesondere während der Schwangerschaft verabreicht. In den meisten Fällen wird Eisen in seiner zweiwertigen Form (Fe²⁺) oral eingesetzt, und zwar insbesondere in Form von Tabletten, Säften etc.

Diese Anwendungsform kann oft zu unerwünschten Nebenreaktionen, wie der kompetitiven Hemmung anderer Medikamente, zu allergischen Reaktionen und Unverträglichkeiten sowie intestinaler Obstipation führen.

Trotz der immensen Fortschritte der Medizin gibt es noch immer Erkrankungen, die zumindest nicht immer erfolgreich therapiert werden können. Zu dieser Gruppe von Krankheiten gehören Tumorerkrankungen. Im Rahmen der vorliegenden Erfindung ist es nun gelungen, ein Therapeutikum für Tumorerkrankungen zur Verfügung zu stellen.

Erfindungsgemäß wird intravenös applizierbares Eisen als alleiniger therapeutischer Wirkstoff zur Herstellung eines Medikamentes zur intravenösen Applikation für die Therapie von Tumorerkrankungen und/oder Infektionskrankheiten verwendet Eisenhaltige Präparate werden bekanntermaßen bei verschiedenen diagnostischen Aufgabenstellungen ebenso vervendet wie bei manchen Therapieformen.

Periodicum Biologorum, Vol.87 (1), 1985, 17-22 beschreibt eine Tumorregession bei experimentell induzierten Tumoren in Mäusen nach intramuskulärer Injektion von Eisen.

Die europäische Patentanmeldung EP-A 0 516 252 beschreibt nanokristalline magnetische Eisenoxid-Partikel, die insbesondere bei der Diagnostik Verwendung finden. Hierbei handelt es sich um Partikel, die aus einem magnetischen Eisenoxidkern und einer an diesen Kern chemisorbierten Hülle bestehen, wobei das Hüllmaterial aus Glycosaminoglycanen besteht. In dieser Patentanmeldung wird vorgeschlagen, derartige Partikel bei der Tumortherapie zu verwenden, wobei sich die Partikel an der Peripherie von Tumoren anreichern sollen, wodurch eine gezielte Strahlen- und/oder Hyperthermietherapie möglich wird.

Die GB-A 1 546 177 beschreibt ein Verfahren zur Herstellung einer injizierbaren stabilen Lösung von Immunoglobulinen und organischen Eisenkomponenten. Das Eisen wird hierbei zur Behandlung von Anämie vorgesehen, wohingegen die Immunoglobuline die Resistenz gegen Infektionserkrankungen erhöhen sollen.

In der europäischen Patentanmeldung EP-A 0 246 734 werden Zusammensetzungen zur Behandlung von Krebs vorgeschlagen, die eine oder mehrere essentielle Fettsäuren enthalten sollen. Die Tumorzellen abtötende Wirkung der Fettsäuren soll in Gegenwart von Eisen verbessert werden.

Eisenpräparate zur intravenösen Applikation sind bereits als Arzneimittel zugelassen. Erfindungsgemäß verwendet werden können solche Präparate, die Eisen in der dreiwertigen Form (Fe³⁺) aufweisen, wobei das Eisen meist in Form von Komplexen vorliegt. In bevorzugter Form handelt es sich hierbei um Natrium-Eisen(III)-gluconat-Komplexe, Eisen(III)-hydroxid-Polymaltose-Komplexe, Ferri-Sorbitol-Citrat-Komplexe oder Ferri-Saccharat-Komplexe. Diese Komplexe sind üblicherweise in Wasser gelöst, wobei Hilfsstoffe wie Benzylalkohol, Puffersubstanzen oder Sorbit vorhanden sein können. Die im Rahmen der vorliegenden Erfindung bevorzugt verwendeten Eisenpräparate beinhalten das Eisen nicht in Form von Partikeln, die beispielsweise aus einem Eisenoxidkern bestehen können, der mit einem Hüllmaterial umgeben ist.

Die erfindungsgemäß bevorzugt eingesetzten Eisenpräparate liegen auch nicht in einer Mischung mit anderen Komponenten, wie beispielsweise Antikörper, Antikörperfragmente u.ä. vor. Es sollte schließlich noch betont werden, daß der erfindungsgemäße Effekt durch das Eisen in den Eisenpräparaten erzielt wird und nicht durch andere Komponenten wie beispielsweise Fettsäuren, die außerdem in anderen Präparaten vorhanden sein können.

Bei der erfindungsgemäßen Verwendung spielt die Dosierung des intravenös applizierbaren Eisens eine wesentliche Rolle. Üblicherweise erhalten die Patienten eine Menge von etwa 100 mg bis 500 mg Eisen pro Woche verabreicht. Dies entspricht etwa einer Menge von 0,5 mg Eisen pro Kilogramm Körpergewicht pro Woche bis zu 5 mg Eisen pro Kilogramm Körpergewicht pro Woche.

Das erfindungsgemäß intravenös applizierbare Eisen wird entweder in Form von Injektionen oder in Form von Dauerinfusionen (i.v.) verabreicht, wobei die intravenöse Injektion bevorzugt ist. In bevorzugter Weise werden Injektionslösungen verwendet, die 20 mg Fe pro ml Injektionslösung enthalten.

In einer weiteren, besonders bevorzugten Ausführungsform wird das intravenös injizierbare Eisen vorher radioaktiv bestrahlt. In einer weiteren bevorzugten Ausführungsform können auch radioaktive Isotope des Eisens wie Eisen 59 oder Eisen 55 verwendet werden.

Besonders betont werden sollte, daß es möglich ist, das injizierbare Eisen als alleinigen therapeutischen Wirkstoff einzusetzen. Die gleichzeitige Behandlung mit anderen Medikamenten, die zur Tumortherapie geeignet sind, ist nicht erforderlich. Es ist allerdings möglich, zur Unterstützung der Wirkung der erfindungsgemäßen Verwendung den Patienten auch einer radioaktiven Bestrahlung zu unterziehen. Die hierbei erzielbaren therapeutischen Wirkungen lassen sich in verschiedenen Fällen durch die begleitende Bestrahlung verbessern.

Die erfindungsgemäße Verwendung des intravenös applizierbaren Eisens wird in erster Linie am Menschen erfolgen. Eine Anwendung an Säugetieren kann vor allem dann in Betracht kommen, wenn es sich um wertvolle Tiere oder um Haustiere handelt, zu denen emotionale Beziehungen bestehen.

Im Rahmen der vorliegenden Erfindung wurde auch gefunden, daß intravenös applizierbares Eisen zur Therapie von Infektionskrankheiten dienen kann. Die Infektionskrankheiten können dabei durch bakterielle Erreger, virale Erreger, mykotische Erreger oder parasitische Erreger hervorgerufen werden. Ein Einsatz von intravenös injizierbarem Eisen scheint insbesondere bei solchen Infektionskrankheiten erfolgversprechend zu sein, die bisher schwierig zu behandeln sind wie Syphillis, Lepra, Pest, Tuberkulose, verschiedene Kinderkrankheiten, AIDS oder Rinderwahnsinn (Kreutzfeld-Jakobsen). Dieser Aspekt hat insbesondere Bedeutung, da bestimmte Tumoren wie die des weißen und lymphatischen Blutbildes eine virale Genese haben sollen (Epstein-Barr-Virus).

Ohne an eine bestimmte Theorie gebunden sein zu wollen, könnte der folgende vorgeschlagene Wirkmechanismus den überraschenden Effekt des erfindungsgemäß verwendeten intravenös injizierbaren Eisens erklären. Aufgrund seiner hohen Turn-over-Rate in der Zellteilung hat der Tumor selbst einen hohen Sauerstoffverbrauch. Die ausreichende Sauerstoffzufuhr ist Voraussetzung für ein rasches Wachstum des Tumors, wobei der Sauerstoff über das Hämoglobin der roten Blutkörperchen zur Verfügung gestellt wird. Tumorgewebe kann aber möglicherweise durch Chemotaxis der Membranen der Erythrocyten im oder am Tumor binden. So kommt es häufig zu blutdurchtränkten Tumorgeweben, in denen die Erythrocyten aufgrund zu langer Verweildauer untergehen. Wenn Eisen sich im Tumorgewebe ablagert, kann es zur Bildung von Nekrosen kommen. Dieser Vorgang könnte wohl ein körpereigener Mechanismus der Tumorbekämpfung sein, der permanent im Körper stattfinden kann und vom Gleichgewicht der beteiligten Kräfte abhängt. Dies würde auch in Übereinstimmung mit der Aussage stehen, daß Vitamin C eine krebsprotektive Wirkung hat, da es in der Atmungskette des Citratzyklus in enger katalytischer Wechselwirkung zum Wertigkeitswechsel von ebenfalls dort vorhandenen II- und III-wertigem Eisen eingebunden ist.

Wenn nun das Eisen bei der erfindungsgemäßen Verwendung intravenös injiziert wird, werden einerseits über das Transferrin das Hämoglobin und Myoglobin völlig mit Eisen gesättigt und das darüber hinaus vorhandene dreiwertige Eisen wird möglicherweise durch ein gestörtes Sauerstoffmilieu im Tumor, wie auch durch die unphysiologisch erhöhte Affinität der Tumorzellenmembram im Krebsgewebe angelagert, das hierdurch sukzessive und spezifisch zerstört wird.

Durch die Injektion von Eisen werden die gesunden Zellen des Patienten immer weiter gestärkt. Nach dem Verschwinden des Tumors und der Metastasen muß allerdings die Verwendung von intravenös injizierbarem Eisen beendet werden, damit keine negativen überschießenden Effekte wie artificielle Hämosidreose oder Nekrosen erzielt werden.

Vorteil der erfindungsgemäßen Verwendung ist, daß jedes Gewebe im Körper erreicht werden kann, wobei unter Ausnutzung der vorgegebenen venösen Blutbahnen das injizierbare Eisen auch Tumore an noch so unzugänglichen Gewebeteilen wie Hirnstamm, Knochenmark etc. erreichen kann und dort seine Wirkung entfalten kann.

Es scheint, daß jede Krebsart durch die erfindungsgemäße Verwendung von intravenös injizierbarem Eisen therapierbar ist, so auch leukämische Formen, da der Entstehungsort aller korpuskulären Blutelemente zu 80 % das Knochenmark ausmacht. Gerade hier könnte es sinnvoll sein - ähnlich der Radio-Jod-Therapie bei Schilddrüsencarzinom bzw. der Radium-Einlagen bei Uteruscarzinomen - entweder den Patienten zu bestrahlen oder das zu injizierende Eisen zu bestrahlen. In besonders schweren Fällen kann ein kombinierter Einsatz dieser beiden Therapien sinnvoll sein. Jeder Radiologe kennt die limitierende Strahlendosis, die sich vorzugsweise am Stand des Blutbildes ausrichtet. Durch die korporale Tumorbestrahlung erfährt das dort angereicherte Blut und mit ihm das injizierte Eisen eine besondere mobile Strahleneigenschaft, die sich bevorzugt wieder an den Metastasen segensreich auswirkt. Der gleiche Effekt wäre bei radioaktivem Eisen zu beobachten, was zur Potenzierung des therapeutischen Effektes führen kann.

Die folgenden Beispiele beschreiben Tumorpatienten, bei denen intravenös applizierbares Eisen erfolgreich zur Therapie der Tumorerkrankungen eingesetzt wurde. Weiterhin wird durch Beispiele die überraschende Wirkung der erfindungsgemäßen Verwendung bei Infektionskrankheiten belegt.

### Beispiel 1

Bei einer weiblichen Patientin wurde im Alter von 85 Jahren ein stenosierendes Adenocarzinom im Colon ascendens diagnostiziert. Die Patientin stimmte einer Operation nicht zu und wurde gegen ärztlichen Rat aus der Klinik entlassen. Über eine Dauer von zwei Jahren wurde injizierbares Eisen intravenös appliziert.

Nach dem Tod der 87-jährigen Patientin wurde eine Obduktion durchgeführt. Dabei wurde festgestellt, daß sich an der Stelle, an der sich der stenosierende Tumor befand, in der Darmwand lediglich eine flache, errosive Veränderung zu erkennen war. Interessanterweise konnte eine Metastasierung des Carzinoms über eine Dauer von mehr als zwei Jahren nicht beobachtet werden.

### Beispiel 2

Bei einer 45-jährigen Patientin wurde ein hoffnungsloses Stadium eines metastasierenden (Leber, Knochen, Lunge, Niere) Leiomyosarkoms in noch ausreichendem Allgemeinzustand mit äußerst schlechter Prognose festgestellt. Es wurde wöchentlich intravenös injizierbares Eisen in einer Menge von 5 ml (jeweils 100 mg Fe) appliziert. Während der Therapie mit intravenös injizierbarem Eisen verschwanden die Lebermetastasen. Nach eineinhalb Jahren wurde die Therapie mit injizierbarem Eisen abgebrochen. Innerhalb von zwei Monaten verstarb die Patientin.

### Beispiel 3

Bei einem 57-jährigen männlichen Patienten wurde bronchoskopisch, histologisch und CT-gesichert ein Plattenepithel-Bronchialcarzinom, rechts, hilusnah, mit ausgedehnten Metastasen u.a. in der Brustwirbelsäule, bei erhöhtem Spontanfrakturrisiko am 4. BWK (Metastase Tischtennisballgröße) diagnostiziert. Unmittelbar nach der Diagnose wurde der gesamte rechte Lungenflügel entfernt und radiologisch nachbehandelt. Das Körpergewicht des Patienten betrug bei Entlassung 95,7 kg.

Nach der Entlassung aus dem Krankenhaus wurde intravenös applizierbares Eisen appliziert und der Patient wurde gleichzeitig einer ambulanten Strahlentherapie in der Klinik unterzogen. In einem Zeitraum von sechs Monaten wurden in regelmäßigen Abständen 80 Injektionen à 5 ml (je 100 mg Fe) Eisen verabreicht und dann die Verabreichung von intravenös applizierbarem Eisen beendet. Zwei Monate nach Beendigung der Eisentherapie konnte röntgenologisch das Verschwinden der erheblichen Wirbelsäulenmetastasen sowie derjenigen Metastasen im Mediastinum und Perikard dokumentiert werden. Darüber hinaus lagen keine Hinweise auf neue Metastasen oder Osteolysen vor.

Nach Beendigung der Eisentherapie betrug das Körpergewicht des Patienten 104 kg, was einer Zunahme von 5 kg entspricht. Der Patient ist als geheilt anzusehen.

### Beispiel 4

Bei einem 61-jährigen männlichen Patienten wurde ein histologisch gesichertes Plattenepithelcarzinom der rechten Nasenrachenregion mit Infiltrationen des gesamten rechten Weichgaumens entdeckt. Eine geplante Radio-Chemo-Therapie lehnte der Patient wegen ärztlich mitgeteilter Aussichtslosigkeit ab. Die voraussichtliche Lebenserwartung bei der Diagnosestellung betrug etwa ein bis zwei Monate.

Der Patient erhielt ambulant wöchentlich drei intravenöse Eiseninjektionen.

Nach etwa fünf Monaten konnten bei einer Kontroll-Computertomographie weder raumfordernde Prozesse, noch Lymphome in der Schädel- bzw. Halsregion festgestellt werden. Der Patient ist diesbezüglich subjektiv beschwerdefrei, kann essen, trinken und behält sein Körpergewicht bei.

### Beispiel 5

Ein 19-jähriger männlicher Patient litt seit der Pubertät an gravider, entstellender Akne conglobata. Hierbei handelt es sich um eine eitrige, durch Streptokokken hervorgerufene Infektion. Durch dermatologische Behandlung konnte die Erkrankung nicht in den Griff bekommen werden und das Ausmaß der Infektion ließ psychische Störungen befürchten. Dem Patienten wurden daraufhin zehnmal hintereinander je einmal pro Woche eine Injektion von Ferrum vitis zu 5 ml verabreicht. Daraufhin verschwand die Akne sukzessive und auf Dauer.

Nach etwa einem Jahr entwickelte derselbe Patient eine nervöse Gastritis, die sich zum Ulcus ventriculi steigerte. Als Erreger dieser Erkrankung wird das Bakterium Helicobacter pylori angesehen.

Nach erneuter zehnmaliger intravenöser Eisenapplikation wurde Helicobacter pylori eliminiert und der Patient war geheilt.

Bei demselben Patienten wurde offensichtlich unbeabsichtigt durch die Eisentherapie eine urologisch gesicherte, bakterieninduzierte Schrumpfnierenbildung links gestoppt. Wegen Insuffizienz der Klappe am linken Blasen-Ostium bildete sich eine Reflux-Niere mit Gefahr der Urosepsis (hervorgerufen beispielsweise durch Coli-Bakterien). Die Niere konnte gerettet werden und die insuffiziente Ureterklappe wurde operativ korrigiert.

### Beispiel 6

Eine 46-jährige Patientin litt an klinisch errosiver Gastritis, die durch Helicobacter-Bakterien verursacht wurde.

Der Patientin wurden einmal wöchentlich 5 ml Eisen intravenös appliziert. Nach einer Behandlungsdauer von 5 Wochen war die Patientin beschwerdefrei und das Helicobacter-Bakterium konnte nicht mehr nachgewiesen werden.

### Beispiel 7

Ein 57-jähriger Patient (Plattenepithel-Ca des Epipharynx) litt an einer kernspintomographisch gesichteten chronischen Osteomyelitis mit Sequeter im rechten proximalen Oberschenkelknochen - nach Fraktur und Operation.

Der anfängliche Einsatz starker Analgetika (Tramal) konnte sukzessive nach intravenöser Eisentherapie eingestellt werden. Hierbei wurden wöchentlich drei Injektionen à 5 ml verabreicht.

Die wegen der Krebserkrankung initiierte Eisentherapie hatte sozusagen als Nebeneffekt die Sanierung des osteomyelitischen Infektionsherdes zur Folge. Dies wurde durch eine Verbesserung der Blutsenkung belegt, die sich innerhalb eines Monats von BKS 18/62 auf BKS 4/30 verbesserte. Eine gezielte Osteomyelitis-Therapie war nicht mehr notwendig, da offensichtlich die dort in der Regel als Problemkeime angesehenen Bakterien auch durch die Eisentherapie vernichtet wurden.

### Beispiel 8

Bei einer 30-jährigen Patientin entwickelte sich eine mit Antibiotika kaum mehr beherrschbare Ovaritis links. Vor der stationären Einweisung wegen abdomineller Abwehrspannung wurde eine intravenöse Eiseninjektionstherapie eingeleitet. Nach fünfmaliger Durchführung der Eiseninjektion wurde die Patientin beschwerdefrei (nach einer Woche).

Bei diesem Fall wurde auch beobachtet, daß die unter Antibiotikatherapie bei Frauen häufig zu beobachtenden Vaginalmykosen unter Eisentherapie nicht auftreten.

### Beispiel 9

Eine 52-jährige Patientin litt seit Jahren an einer chronischen Erkrankung, die durch Herpesviren hervorgerufen wurde. Hierbei handelte es sich um Herpes facialis, Herpes nasalis und Herpes labialis. Während einer akuten, schmerzenden Phase eines Lippenherpes wurde Eisen intravenös appliziert. Über eine Dauer von 10 Wochen wurden wöchentlich jeweils 5 ml Eisen appliziert. Ohne sonst übliche Zusatzmedikationen bildete sich das Herpesvirus zurück und trat auf Dauer nicht mehr auf.

### Beispiel 10

Eine 41-jährige Patientin entwickelte unter Streß ein Ulcusduodeni-Recidiv. Eine Eigenmedikation mit Antiacida blieb auf Dauer erfolglos. Daraufhin erhielt die Patientin 5 ml Ferrum vitis intravenös in nicht ganz regelmäßigen Abständen. Bereits nach der dritten Injektion ebbten die Schmerzen ab und verloren sich ganz auf Dauer nach Fortführung der nur punktuellen Eisentherapie. Offensichtlich gelangt das intravenös applizierbare Eisen auch über die Blutwege an die Erreger (Helicobacter) und kann diese eliminieren.

## Patentansprüche

1. Verwendung von intravenös applizierbarem Eisen als alleiniger therapeutischer Wirkstoff zur Herstellung eines Medikaments geeignet für die intravenöse Applikation für die intravenöse Therapie von Tumorerkrankungen und/oder Infektionskrankheiten.

2. Verwendung nach Anspruch 1, dadurch gekennzeichnet, daß das Medikament für eine Dosierung des intravenös applizierbaren Eisens von 100 mg bis 500 mg Fe pro Woche angepaßt ist.

3. Verwendung nach einem der Ansprüche 1 oder 2, dadurch gekennzeichnet, daß das intravenös applizierbare Eisen vorher radioaktiv bestrahlt wurde.

4. Verwendung nach einem der Ansprüche 1 bis 3, dadurch gekennzeichnet, daß das Medikament so angepaßt ist, daß das intravenös applizierbare Eisen der alleinige therapeutische Wirkstoff ist und der mit dem Medikament behandelte Patient darüber hinaus lediglich durch radioaktive Bestrahlung behandelt wird.

## Claims

1. Use of intravenously administered iron as the sole therapeutic active ingredient in the preparation of a medicament suitable for intravenous administration for the intravenous treatment of tumorous diseases and/or infectious diseases.

2. Use according to claim 1, characterised in that the medicament is made up for a dosage of the intravenously administered iron of from 100 mg to 500 mg per week.

3. Use according to one of claims 1 or 2, characterised in that the intravenously administered iron is radioactively irradiated beforehand.

4. Use according to one of claims 1 to 3, characterised in that the medicament is made up in such a way that the intravenously administered iron is the sole therapeutic active ingredient and the patient treated with the medicament is besides this treated only by radioactive radiation.

## Revendications

1. Utilisation de fer administrable par voie intraveineuse comme seul et unique agent thérapeutique actif pour la préparation d'un médicament approprié à l'administration intraveineuse pour la thérapie intraveineuse d'affections tumorales et/ou de maladies infectieuses.

2. Application selon la revendication 1, caractérisée en ce que le médicament est adapté pour un dosage de fer administrable par voie intraveineuse de 100 mg à 500 mg Fe par semaine.

3. Application selon l'une des revendications 1 ou 2, caractérisée en ce que le fer administrable par voie intraveineuse est préalablement irradié par une source radioactive.

4. Application selon l'une des revendications 1à 3, caractérisée en ce que le médicament est adapté de manière que le fer administrable par voie intraveineuse constitue le seul agent thérapeutique actif et que le patient traité par le médicament ne soit complémentairement traité que par irradiation radioactive.
